# EUROPÄISCHE PATENTSCHRIFT

(11) **EP 0 801 130 B1**
(45) Veröffentlichungstag und Bekanntmachung des Hinweises auf die Patenterteilung: **05.02.2003**
(21) Anmeldenummer: 97102847.7
(22) Anmeldetag: 21.02.1997
(51) Int. Cl.: C11D 1/66, C11D 17/00, C11D 3/20, C11D 3/43, A61K 7/50

(54) **Tensidhaltige Reinigungsmittel in Form einer Mikroemulsion**
Surfactant containing microemulsion cleaning composition
Composition de nettoyage sans forme de microémulsion contenant des agents tensioactifs

(30) Priorität: 18.04.1996 DE 19615271
(43) Veröffentlichungstag der Anmeldung: 15.10.1997
(73) Patentinhaber: SASOL Germany GmbH, 22297 Hamburg (DE)
(72) Erfinder: Balzer, Dieter, Dr., 45721 Haltern (DE)
(74) Vertreter: Schupfner, Gerhard D., Dr. Dipl.-Chem.

(56) Entgegenhaltungen:
- EP-A- 0 280 143
- EP-A- 0 507 047
- WO-A-90/08206
- WO-A-92/07543
- WO-A-95/31957
- DE-A- 19 530 220

## Beschreibung

Die Erfindung betrifft milde tensidhaltige Mittel in Form von Mikroemulsionen zur Reinigung der Haut sowie harter Oberflächen.

Während die Verwendung von Makroemulsionen als Reinigungsmittel für praktisch alle Oberflächen seit langem bekannt ist, werden entsprechende Mikroemulsionen erst seit kurzem beschrieben. Im Gegensatz zu thermodynamisch metastabilen Makroemulsionen sind Mikroemulsionen Gleichgewichtssysteme, also völlig stabil. Basierend auf der hohen Feinteiligkeit des Systems wird seine freie Energie infolge der starken Zunahme der Dispersionsentropie trotz der endlichen, wenn auch sehr kleinen, Grenzflächenspannung negativ, und damit das System stabil. Eine Folge der Feinteiligkeit, typisch sind ca. 10 nm (5 bis 100 nm) im Durchmesser statt ≥ 1 um wie bei Makroemulsionen, ist die Transparenz bzw. Transluzenz der Flüssigkeiten. Mikroemulsionen vermitteln daher häufig den Eindruck echter Lösungen. Sie bestehen aus den beiden miteinander nicht mischbaren Flüssigkeiten, in der Regel Wasser und einer apolaren Flüssigkeit, dem Tensid oder Tensidgemisch und meist einem Cosolvens zuweilen auch Cotensid genannt. Typische Cosolvenzien sind niedere Alkohole mit 3 bis 5 Kohlenstoffatomen Ihre Wirkung besteht einerseits in der Absenkung der Grenzflächenspannung zwischen den beiden Flüssigkeiten zusätzlich zu der des Tensids und außerdem der Modellierung des Grenzflächenfilms (vgl. D. Langevin, Mol. Cryst. Liquid Cryst 1986, 138, 259-305).

Mikroemulsionen als Reinigungssysteme werden seit Mitte der 80er-Jahre beschrieben So beanspruchen die Patentanmeldungen EP-A-0 137 616 und EP-A-0 160 762 flüssige Vollwaschmittel in Form einer O/W-Mikroemulsion, bestehend aus Terpenen, Paraffinen, Alkylaromaten und/oder halogenierten Kohlenwasserstoffen als fettlösende Lösemittel, Wasser, üblichen Tensiden insbesondere auf petrochemischer Grundlage, Elektrolyten und Aminen als Stabilisatoren. Reiniger für harte Oberflächen in Form von Mikroemulsionen beanspruchen EP 0 316 726, EP 0 368 146, EP 0 620 271 und DE 37 16 526. Sie beinhalten übliche Tenside, Cosolvenzien wie wasserlösliche Alkohole, Polypropylenglykole, Monoalkylether von Glykolen, aliphatische Carbonsäuren oder Phosphorsäureester neben Parfüm und/oder Kohlenwasserstoffen als apolare Flüssigkeiten und ggf. Elektrolyt. Zu erwähnen sind schließlich die beiden Dokumente EP 0 478 086 und WO 92/03528, die spezielle Reinigungsmittel mit desinfizierenden bzw. abrasiven Eigenschaften für harte Oberflächen in Form von Mikroemulsionen beschreiben. Sie basieren auf üblichen, überwiegend petrochemischen Tensiden und üblichen Cosolvenzien, wobei das zuletzt genannte Dokument neben vielen anderen Tensidtypen auch Alkylpolyglucoside benennt.

Die Verwendung der Alkylpolyglucoside als Basistensid bei Mikroemulsionen ist seit einigen Jahren bekannt (vgl. H. Lüders und D. Balzer, Proc. 2nd World Surfactant Congr 1988, Paris, Vol II, 81-93). Allerdings war diese Eigenschaft im Gegensatz etwa zu Fettalkoholoxethylaten auch immer an die Verwendung von üblichen Cosolvenzien wie C₃-C₆-Alkohole, Alkylglykole, Alkyloligoglykole, Amine, etc. gebunden, die allesamt geruchlich nicht akzeptabel und/oder toxikologisch bedenklich sind, so daß eine Anwendung dieser Mikroemulsionssysteme als manuelle Reinigungsflüssigkeit kaum in Frage kommt, ganz zu schweigen von kosmetischen Anwendungen. Im Hinblick auf letztere sind jedoch Alkylpolyglucoside besonders interessant. So haben humantoxikologische Tests (DKT) ergeben, daß diese im Vergleich zu anderen in der Kosmetik häufig angewendeten Tensiden besonders hautfreundlich sind. Dies dürfte an der geringeren Entfettungswirkung liegen etwa im Vergleich zu Fettalkoholethersulfaten - wie Entfettungsversuche an mit radioaktiv markiertem Sebum belegter Schweinehaut ergaben. Diese stärkere Hautschonung wird auch bei Mischungen von C₁₀C₁₂- bzw. C₁₂C₁₄-Alkylpolyglucosiden mit Ethersulfaten erreicht, wie die gegenüber reinem Ethersulfat weit höhere Restradioaktivität anzeigt. Folglich sollten sich Alkylpolyglycoside auch in Form von wäßrigen Mikroemulsionen insbesondere für kosmetische Anwendungen nutzen lassen. Analoges gilt für alle Reinigungsprozesse, bei denen die Hautoberfläche mit der Reinigungsflüssigkeit in Berührung kommen kann Diese Aufgabenstellung impliziert aber, daß es gelingt, geeignete Cosolvenzien zu finden, die nicht die obenerwähnten Nachteile haben, sondern toxikologisch unbedenklich und geruchlich zumindest neutral sind.

Die Aufgabe wird gelöst durch die erfindungsgemäße Kombination aus Tensid und Cosolvens. Danach lassen sich überraschend Mikroemulsionen mit hohen Öl- und Wassergehalten durch Alkylpolyglycoside, ggf in Kombination mit geringen Anteilen anderer Tenside, und Estern mehrbasischer Carbon- oder Hydroxycarbonsäuren mit C₁bis C₄-Alkoholen als Cosolvenzien herstellen.

Gegenstand der Erfindung ist daher ein Reinigungsmittel zur Verwendung zur Reinigung der Haut oder der manuellen Reinigung harter Oberflächen, in Form einer Mikroemulsion bestehend im wesentlichen aus Öl, Wasser, Tensid und Cosolvens, wobei das Tensidsystem 80-100 % Alkylpolyglycoside und 0 - 20 % Hilfstentenside enthält und das Cosolvens aus Oligoestern mehrbasischer Carbon- oder Hydroxycarbonsäuren mit Alkoholen mit 1 bis 4 Kohlenstoffatomen sowie deren Gemischen besteht.

Die EP-A-0 845 987 hat transluzente Antitranspirantien zum Gegenstand. Die EP-A-0 845 987 nimmt die Priorität der DE-A-195 30 220 in Anspruch und ist Stand der Technik gemäß Art. 54(3) EPÜ. Die EP-A-0 845 987 offenbart Mikroemulsionen zur Verwendung in Antitranspirantien. Eine der offenbarten Mikroemulsionen besteht aus 26 Gew.-% Plantaren® 2000, 20 Gew.-% Dioctylether, 5 Gew.-% Parfümöl, 39 Gew.-% Wasser und 10 Gew.-% Triethylcitrat. Plantaren® 2000 ist ein C8-C16-Alkyl-oligoglycosid der Firma Henkel Corp.. Letztgenannte Mikroemulsion wird daher in den vorliegenden Sachansprüchen ausgenommen.

Ein weiterer Gegenstand der Erfindung ist die Verwendung des Reinigungsmittels zur Reinigung der Haut sowie zur manuellen Reinigung harter Oberflächen.

Die erfindungsgemäßen Mikroemulsionen enthalten 0,3 bis 50 % ölige Phase, 1 bis 60 % Tensid, 0,5 bis 50 % Cosolvens sowie 15 bis 97 % Wasser. Bevorzugt werden ein Gehalt an Öl von 0,5 bis 45 %, an Tensid von 3 bis 55 %, an Gosolvens von I bis 45 % und an Wasser von 18 bis 95 %. Besonders bevorzugt sind Gehalte an 01 von 1 bis 40 %, an Tensid von 5 bis 50 %, an Gosolvens von 3 bis 40 % und an Wasser von 20 bis 93%.

### Öle

Als Ölphasen kommen insbesondere die in der Kosmetik eingesetzten Öle in Frage wie z. B. natürliche und synthetische Triglyceride der verschiedenen Fettsäuren sowie deren Di- und Monoglyceride, Fettsäureester niederer Alkohole wie Isopropylfettsäureester, Paraffinöle, Ricinusöl, sowie deren Gemische, ggf mit bis zu 20 % Zusätzen an Fettalkoholen, Terpenen und anderen Geruchsstoffen, sowie Silikonölen und deren Mischungen.

### Tenside

Die erfindungsgemäß eingesetzten Alkylpolyglycoside genügen der Formel I

R-O-Zₙ (I),

in der R für einen linearen oder verzweigten, gesättigten oder ungesättigten aliphatischen Alkylrest mit 8 bis 16 Kohlenstoffatomen oder Gemische davon und Zₙ für einen Polyglycosylrest mit n = 1,0 bis 3 Hexose- oder Pentoseeinheiten oder Gemische davon steht.

Bevorzugt werden Alkylpolyglycoside mit einem Polyglycosylrest mit n = 1,1 bis 2 Glycosyleinheiten, ganz besonders bevorzugt mit n = 1,1 bis 1,6 Glycosyleinheiten. Bevorzugte Alkylpolyglycoside sind Alkylpolyglucoside.

Die Alkylpolyglycoside können nach bekannten Verfahren auf Basis nachwachsender Rohstoffe hergestellt werden. Beispielsweise wird Dextrose in Gegenwart eines sauren Katalysators mit n-Butanol zu Butyl(poly)glucosidgemischen umgesetzt, welche mit langkettigen Alkoholen ebenfalls in Gegenwart eines sauren Katalysators zu den gewünschten Alkylpolyglucosidgemischen umglycosidiert werden. Oder Dextrose wird unmittelbar mit dem gewünschten langkettigen Alkohol umgesetzt.

Die Struktur der Produkte ist in bestimmten Grenze variierbar. Der Alkylrest R wird durch die Auswahl des langkettigen Alkohols festgelegt. Günstig aus wirtschaftlichen Gründen sind die großtechnisch zugänglichen Tensidalkohole mit 8 bis 16 C-Atomen, insbesondere native Fettalkohole aus der Hydrierung von Fettsäuren bzw. Fettsäurederivaten. Verwendbar sind auch Ziegleralkohole oder Oxoalkohole.

Der Polyglycosylrest Zₙ wird einerseits durch die Auswahl des Kohlenhydrats und andererseits durch die Einstellung des mittleren Polymerisationsgrads n z. B. nach DE-OS 19 43 689 festgelegt. Im Prinzip können Polysaccharide wie z. B. Stärke oder Maltodextrine und Dextrose, etc. eingesetzt werden. Bevorzugt wird die großtechnisch leicht zugängliche Dextrose verwendet. Da die wirtschaftlich interessanten Alkylpolyglycosidsynthesen nicht regio- und stereoselektiv verlaufen, sind Alkylpolyglycoside stets Gemische von Oligomeren, die ihrerseits Gemische verschiedener isomerer Formen darstellen. n ist ein Mittelwert, der daher auch nicht ganzzahlig sein kann. Die Alkylpolyglycoside liegen nebeneinander mit α- und β-glycosidischen Bindungen in Pyranose- und Furanoseform vor. Auch die Verknüpfungsstellen zwischen zwei Saccharidresten sind unterschiedlich.

Erfindungsgemäß eingesetzte Alkylpolyglycoside lassen sich auch durch Abmischen von Alkylpolyglycosiden mit Alkylmonoglycosiden herstellen. Letztere kann man z. B. nach EP-A-0 092 355 mittels polarer Lösemittel wie Aceton aus Alkylpolyglycosiden gewinnen bzw. anreichern.

Der Glycosidierungsgrad kann zweckmäßigerweise mittels Hochtemperaturgaschromatographie der silylierten Alkylpolyglycoside bestimmt werden.

Ökologisch zählen die Alkylpolyglycoside zu den umweltfreundlichsten Tensiden. So wurden bei Prüfung der biologischen Abbaubarkeit (Couplet Unit Test, DOC-Messung) an C₁₀C₁₂- und an C₁₂C₁₄-Alkylpolyglucosiden Werte von 95 bis 97 % erreicht.

Die Toxizitätsdaten mit LD 50 (Ratte > 10 000 mg/kg, LC 50 (Goldorfe) 12 - 40 mg/l und EC 50 (Daphnia) 30 - 110 mg/l bei je 2 C₁₀C₁₂- bzw. C₁₂C₁₄-Alkylpolyglucosiden deuten ebenfalls ein im Vergleich mit vielen anderen Tensiden, auch üblichen Silikonentschäumerdispergatoren, hervorragendes Umweltverhalten an.

In manchen Fällen kann es zweckmäßig sein, das Alkylpolyglycosid mit bis zu 20 %, vorzugsweise bis zu 15 %, besonders bevorzugt bis zu 10 %, bezogen auf die Tensidgesamtmenge, Hilfstensid zu kombinieren. Als Hilfstenside sind anionische Tenside wie C₈-C₁₈-Fettalkoholsulfate, C₈-C₁₈-Fettalkoholethersulfate mit 1 bis 3 Mol Ethylenoxid/mol, carboxymethylierte C₁₀-C₁₈-Fettalkoholoxethylate mit 3 bis 10 Mol Ethylenoxid/mol, ethoxylierte C₁₀-C₁₈-Fettalkoholsulfosuccinate mit 2 bis 6 mol Ethylenoxid/mol, nichtionische Tenside wie C₁₀-C₁₈-Oxethylate mit 3 bis 50 mol Ethylenoxid/mol, C₈-C₁₄-Fettsäure N-Alkylglucamide, ethoxylierte und nicht ethoxylierte Sorbitanester vom Tween- bzw. Span-Typ, sowie betainische Tenside vom Typ der Alkylamidopropylbetaine sowie deren Gemische geeignet.

### Cosolvenzien

Erfindungsgemäße Cosolvenzien sind Oligoester mehrbasischer Carbonsäuren und/oder Hydroxycarbonsäuren mit niederen Alkoholen (C₁-C₄). Bevorzugt sind Ester von mehrbasischen C₂ bis C₆-Carbon- oder Hydroxycarbonsäuren. Besonders bevorzugt sind die Ester der mehrbasischen C₂ bis C₅-Carbonsäuren mit C₂ bis C₄-Alkoholen. Typische Cosolvenzien sind z. B. Diethyltartrat, Di-isopropyltartrat, Di-n-propyltartrat, Di-butyltartrate, Triethylcitrat, etc.

### Weitere Bestandteile

Weitere Bestandteile der Mikroemulsion neben Wasser sind ggf. Elektrolyte, Farbstoffe, Trübungsmittel, Konservierungsmittel, etc.

Die Herstellung der erfindungsgemäßen wäßrigen Mikroemulsionen ist im allgemeinen unproblematisch, da es sich im Gegensatz zu Makroemulsionen um Gleichgewichtssysteme handelt Ein Weg zu ihrer Herstellung besteht z. B. darin, Ölphase, Cosolvens und Tenside zunächst unter Rühren zu vereinigen und so dann mit Wasser zu verdünnen. Das Öl-Wasserverhältnis sollte zwischen 1 : 1 000 bis 3 : 1, vorzugsweise zwischen 1 : 500 bis 2 : 1 liegen und für das Tenside-Cosolvens-Verhältnis gilt 100 : 1 bis 1 : 3, vorzugsweise 50 : 1 bis 1 : 2.

Nicht unwesentlich ist der pH-Wert der Reinigungsmittel, der im neutralen Bereich zwischen 5 und 8 liegen sollte.

### Beispiele

Die nachstehenden erfindungsgemäßen Beispiele sollen das Verfahren erklären (Tabelle 1). Die im wesentlichen wasserklaren und dünnflüssigen Flüssigkeiten zeigten in dem hinsichtlich ihrer Lagerung bzw. Anwendung relevanten Temperaturbereich (5 bis 50 °C) auch nach 3 Monaten keinerlei Veränderungen. Sie ließen sich mit Wasser in weiten Grenzen verdünnen

In Berührung mit der menschlichen Haut vermittelten die Flüssigkeiten auch nach wiederholter Anwendung ein angenehmes Gefühl. Manuelle Reinigungstests an künstlich angeschmutzten Fliesen (Modellschmutz aus 20 % Motoröl, 20W50, 20 % Getriebeöl SAE90, 10 % Schmierfett DIN 51 818, Klasse 2, 10 % Seesand fein, 0,5 % Benton® 34, 30 % Eisenoxid schwarz und 9,5 % Farbruß CK3, Degussa) zeigen im Vergleich zu Marktprodukten eine überlegene Reinigungswirkung.

| | 1 | 2 | 3 | 4 | 5 | 6 | 7 | 8 | 9 |
|---|---|---|---|---|---|---|---|---|---|
| Öle | | | | | | | | | |
| Isopropylmyristat | 35 | 33 | 28 | 7,5 | 5 | 0,5 | 7,5 | 15 | 25 |
| Paraffinöl * | -- | 2 | -- | 0,5 | -- | *--* | *--* | *--* | -- |
| Miglyol® 812 ** | -- | -- | 5 | -- | -- | -- | -- | -- | -- |
| Fichtennadelöl *** | -- | -- | -- | 7,5 | -- | -- | 7,5 | -- | -- |
| Orangenterpen *** | -- | -- | 2 | -- | -- | -- | -- | 1 | -- |

| Tenside | | | | | | | | | |
|---|---|---|---|---|---|---|---|---|---|
| C₁₂C₁₄Alkylpolyglucosid_{1.2} | 25 | 25 | 25 | 35 | 22,5 | 2,25 | 30 | 35 | 25 |
| C₈C₁₀Alkylpolyglucosid_{1.2} | -- | -- | -- | -- | 22,5 | 2,25 | -- | -- | -- |
| C₁₂C₁₄(EO)₇H | -- | -- | -- | -- | -- | -- | 5 | -- | -- |
| C₁₂C₁₄(EO)₄CH₂COONa | -- | -- | -- | -- | -- | -- | -- | 3 | -- |

| Cosolvenzien | | | | | | | | | |
|---|---|---|---|---|---|---|---|---|---|
| Diethyltartrat | 15 | 15 | 15 | -- | -- | -- | -- | -- | -- |
| Triethylcitrat | -- | -- | -- | 15 | 4,5 | 0,45 | 15 | 10 | 25 |
| Di-propylsuccinat | -- | -- | -- | -- | -- | -- | -- | 3 | -- |
| Wasser | ad | | | | | | | | |

| | | | | | | | | | |
|---|---|---|---|---|---|---|---|---|---|
| * DAB | | | | | | | | | |
| ** Capryl-caprinsäure-triglycerid (Hüls) | | | | | | | | | |
| *** Dragoco | | | | | | | | | |

## Patentansprüche

1. Reinigungsmittel zur Reinigung der Haut oder der manuellen Reinigung harter Oberflächen in Form einer Mikroemulsion bestehend im wesentlichen aus Öl, Wasser, einem Tensidsystem und Cosolvens, wobei
- das Tensidsystem 80 bis 100 % Alkylpolyglycosid und
- 0 bis 20 % Hilfstensid enthält und
- das Cosolvens aus Oligoestern mehrbasischer Carbon- oder Hydroxycarbonsäuren mit Alkoholen mit 1 bis 4 Kohlenstoffatomen sowie deren Gemischen besteht; und
wobei die Mikroemulsion nicht aus einem Gemisch von 26 Gew.-% Plantaren® 2000 (ein C8-C16-Alkyl-oligo-glycosid der Firma Henkel Corp.), 20 Gew.-% Dioctylether, 5 Gew.-% Parfümöl, 39 Gew.-% Wasser und 10 Gew.-% Triethylcitrat besteht.

2. Reinigungsmittel nach Anspruch 1, **dadurch gekennzeichnet, daß** das Alkylpolyglycosid der Formel I
R-O-Z (I),
entspricht, worin R für einen linearen oder verzweigten, gesättigten oder ungesättigten Alkylrest mit 8 bis 16 C-Atomen und Z für einen Oligoglycosidrest steht, und n im Mittel 1 bis 3 bedeutet.

3. Reinigungsmittel nach den Ansprüchen 1 oder 2, **dadurch gekennzeichnet, daß** das Alkylpolyglycosid ein Alkylpolyglucosid ist.

4. Reinigungsmittel nach einem der Ansprüche 1 bis 3, **dadurch gekennzeichnet, daß** das Alkylpolyglycosid einen mittleren Glycosidierungsgrad von 1,1 bis 1,6 hat.

5. Reinigungsmittel nach einem der Ansprüche 1 bis 4, **dadurch gekennzeichnet, daß** das Tensidsystem 0 bis 20 % nichtionische, anionische und/oder betainische grenzflächenaktive Stoffe enthält.

6. Reinigungsmittel nach einem der Ansprüche 1 bis 5, **dadurch gekennzeichnet, daß** das Öl der Mikroemulsion aus natürlichen und/oder synthetischen Triglyceriden der Fettsäuren, Di- oder Monoglyceriden der Fettsäuren, Fettsäureester niederer Alkohole, Paraffinöl, Rizinusöl sowie deren Gemische besteht.

7. Reinigungsmittel nach einem der Ansprüche 1 bis 6, **dadurch gekennzeichnet, daß** das Cosolvens ein Ester von C₂- bis C₄-Alkoholen oder deren Mischungen ist.

8. Reinigungsmittel nach einem der Ansprüche 1 bis 7, **dadurch gekennzeichnet, daß** die Mikroemulsion 0.3 bis 50 % ölige Phase, 1 bis 60 % Tenside, 0,5 bis 50 % Cosolvens sowie 15 bis 97 % Wasser enthält.

9. Reinigungsmittel nach einem der Ansprüche 1 bis 8, **dadurch gekennzeichnet, daß** die Mikroemulsion 1 bis 40 % ölige Phase, 5 bis 50 % Tenside, 3 bis 40 % Cosolvens sowie 20 bis 93 % Wasser enthält.

10. Reinigungsmittel nach einem der Ansprüche 1 bis 9, **dadurch gekennzeichnet, daß** das Öl-Wasser-Verhältnis zwischen 1: 1 000 und 3: 1 und das Tenside Cosolvens-Verhältnis zwischen 100 : 1 und 1: 3 liegt

11. Verwendung eines Reinigungsmittels zur Reinigung der Haut, wobei das Reinigungsmittel in Form einer Mikroemulsion vorliegt bestehend im wesentlichen aus Öl, Wasser, einem Tensidsystem und Cosolvens, wobei
- das Tensidsystem 80 bis 100 % Alkylpolyglycosid und
- 0 bis 20 % Hilfstensid enthält und
- das Cosolvens aus Oligoestern mehrbasischer Carbon- oder Hydroxycarbonsäuren mit Alkoholen mit 1 bis 4 Kohlenstoffatomen sowie deren Gemischen besteht.

12. Verwendung eines Reinigungsmittels zur manuellen Reinigung harter Oberflächen, wobei das Reinigungsmittel in Form einer Mikroemulsion vorliegt bestehend im wesentlichen aus Öl, Wasser, einem Tensidsystem und Cosolvens, wobei
- das Tensidsystem 80 bis 100 % Alkylpolyglycosid und
- 0 bis 20 % Hilfstensid enthält und
- das Cosolvens aus Oligoestern mehrbasischer Carbon- oder Hydroxycarbonsäuren mit Alkoholen mit 1 bis 4 Kohlenstoffatomen sowie deren Gemischen besteht.

## Claims

1. A cleaning agent in form of a microemulsion for cleaning the skin or manually cleaning hard surfaces, consisting essentially of oil, water, a surfactant system and cosolvent, wherein
- the surfactant system comprises 80 to 100 % of alkyl polyglycoside and
- 0 to 20 % of an auxiliary surfactant and
- the cosolvent consists of oligoesters of poly-basic carboxylic or hydroxycarboxylic acids with alcohols having from 1 to 4 carbon atoms and mixtures thereof; and
wherein the microemulsion does not consist of a mixture of 26 wt.% of Plantaren® 2000 (a C₈-C₁₆ alkyl-oligoglycoside of Henkel Corp.), 20 wt.% of dioctyl ether, 5 wt.% of perfume oil, 39 wt.% of water, and 10 wt.% of triethyl citrate.

2. The cleaning agent according to claim 1, **characterized in that** the alkyl polyglycoside corresponds to formula I
**R-O-Z (I),**
where **R** represents a linear or branched, saturated or unsaturated alkyl residue having from 8 to 16 carbon atoms, and **Z** is an oligoglycoside residue, and **n** means an average of from 1 to 3.

3. The cleaning agent according to any one of claims 1 or 2, **characterized in that** the alkyl polyglycoside is an alkyl polyglucoside.

4. The cleaning agent according to any one of claims 1 to 3, **characterized in that** the alkyl polyglycoside has an average glycosidation degree of from 1.1 to 1.6.

5. The cleaning agent according to any one of claims 1 to 4, **characterized in that** the surfactant system contains 0 to 20 % of nonionic, anionic, and/or betainic surface-active substances.

6. The cleaning agent according to any one of claims 1 to 5, **characterized in that** the oil of the microemulsion consists of natural and/or synthetic triglycerides of the fatty acids, di- or monoglycerides of the fatty acids, fatty acid esters of lower alcohols, paraffin oil, castor oil, and the mixtures thereof.

7. The cleaning agent according to any one of claims 1 to 6, **characterized in that** the cosolvent is an ester of C₂- to C₄-alcohols or mixtures thereof.

8. The cleaning agent according to any one of claims 1 to 7, **characterized in that** the microemulsion contains 0.3 to 50 % of oily phase, 1 to 60 % of surfactants, 0.5 to 50 % of cosolvent, and 15 to 97 % of water.

9. The cleaning agent according to any one of claims 1 to 8, **characterized in that** the microemulsion contains 1 to 40 % of oily phase, 5 to 50 % of surfactants, 3 to 40 % of cosolvent, and 20 to 93 % of water.

10. The cleaning agent according to any one of claims 1 to 9, **characterized in that** the oil-water ratio is from 1 : 1,000 to 3 : 1 and the surfactant-cosolvent ratio is from 100 : 1 to 1 : 3.

11. Use of a cleaning agent in form of a microemulsion for cleaning the skin, consisting essentially of oil, water, a surfactant system and cosolvent, wherein
- the surfactant system comprises 80 to 100 % of alkyl polyglycoside and
- 0 to 20 % of an auxiliary surfactant and
- the cosolvent consists of oligoesters of polybasic carboxylic or hydroxycarboxylic acids with alcohols having from 1 to 4 carbon atoms and mixtures thereof.

12. Use of a cleaning agent in form of a microemulsion for manually cleaning hard surfaces consisting essentially of oil, water, a surfactant system and cosolvent, wherein
- the surfactant system comprises 80 to 100 % of alkyl polyglycoside and
- 0 to 20 % of an auxiliary surfactant and
- the cosolvent consists of oligoesters of polybasic carboxylic or hydroxycarboxylic acids with alcohols having from 1 to 4 carbon atoms and mixtures thereof.

## Revendications

1. Produit nettoyant destiné au nettoyage de la peau ou au nettoyage manuel de surfaces dures, sous forme d'une micro-émulsion contenant principalement une huile, de l'eau, un système tensio-actif et un co-solvant, dans lequel
- le système tensio-actif contient 80 à 100 % d'alkylpolyglycoside et
- 0 à 20 % de tensio-actif auxiliaire, et
- le co-solvant est constitué d'oligo-esters d'acides carboxyliques ou hydroxycarboxyliques polyfonctionnels avec des alcools ayant 1 à 4 atomes de carbone, ainsi que leurs mélanges ;
et dans lequel la micro-émulsion n'est pas constituée d'un mélange de 26 % en poids de Plantaren® 2000 (alkyloligo-glycoside en C₈ à C₁₆ de la firme Henkel Corp.), 20 % en poids d'éther de dioctyle, 5 % en poids d'huile essentielle, 39 % en poids d'eau et 10 % en poids de citrate de triéthyle.

2. Produit nettoyant suivant la revendication 1, **caractérisé en ce que** l'alkylpolyglycoside répond à la formule I
R-O-Z (I)
dans laquelle R représente un reste alkyle linéaire ou ramifié, saturé ou non saturé, ayant 8 à 16 atomes de carbone et Z est un reste oligoglycoside, et n a une valeur moyenne de 1 à 3.

3. Produit nettoyant suivant les revendications 1 ou 2, **caractérisé en ce que** l'alkylpolyglycoside est un alkylpolyglucoside.

4. Produit nettoyant suivant l'une des revendications 1 à 3, **caractérisé en ce que** l'alkylpolyglycoside a un degré moyen de glycosylation de 1,1 à 1,6.

5. Produit nettoyant suivant l'une des revendications 1 à 4, **caractérisé en ce que** le système tensio-actif contient 0 à 20 % de substances tensio-actives non ioniques, anioniques et/ou bétaïniques.

6. Produit nettoyant suivant l'une des revendications 1 à 5, **caractérisé en ce que** l'huile de la micro-émulsion est constituée de triglycérides naturels et/ou synthétiques des acides gras, de di- ou monoglycérides des acides gras, d'esters d'acides gras d'alcools inférieurs, d'huile de paraffine, d'huile de ricin ainsi que de leurs mélanges.

7. Produit nettoyant suivant l'une des revendications 1 à 6, **caractérisé en ce que** le co-solvant est un ester d'alcools en C₂ à C₄ ou de leurs mélanges.

8. Produit nettoyant suivant l'une des revendications 1 à 7, **caractérisé en ce que** la micro-émulsion contient 0,3 à 50 % de phase huileuse, 1 à 60 % d'agents tensio-actifs, 0,5 à 50 % de co-solvant ainsi que 15 à 97 % d'eau.

9. Produit nettoyant suivant l'une des revendications 1 à 8, **caractérisé en ce que** la micro-émulsion contient 1 à 40 % de phase huileuse, 5 à 50 % d'agents tensio-actifs, 3 à 40 % de co-solvant ainsi que 20 à 93 % d'eau.

10. Produit nettoyant suivant l'une des revendications 1 à 9, **caractérisé en ce que** le rapport de l'huile à l'eau se situe entre 1:1000 et 3:1 et le rapport des agents tensio-actifs au co-solvant se situe entre 100:1 et 1:3.

11. Utilisation d'un produit nettoyant pour le nettoyage de la peau, dans laquelle le produit nettoyant se présente sous forme d'une micro-émulsion principalement constituée d'huile, d'eau, d'un système tensio-actif et d'un co-solvant, où
- le système tensio-actif contient 80 à 100 % d'alkyl-polyglycoside et
- 0 à 20 % d'agent tensio-actif auxiliaire, et
- le co-solvant est constitué d'oligo-esters d'acides carboxyliques ou hydroxycarboxyliques polyfonctionnels avec des alcools ayant 1 à 4 atomes de carbone, ainsi que leurs mélanges.

12. Utilisation d'un produit nettoyant pour le nettoyage manuel de surfaces dures, dans laquelle le produit nettoyant se présente sous forme d'une micro-émulsion constituée principalement d'huile, d'eau, d'un système tensio-actif et d'un co-solvant, où
- le système tensio-actif contient 80 à 100 % d'alkyl-polyglycoside et
- 0 à 20 % d'agent tensio-actif auxiliaire, et
- le co-solvant est constitué d'oligo-esters d'acides carboxyliques ou hydroxycarboxyliques polyfonctionnels avec des alcools ayant 1 à 4 atomes de carbone, ainsi que leurs mélanges.
